# EUROPÄISCHE PATENTSCHRIFT

(11) **EP 2 021 806 B1**
(45) Veröffentlichungstag und Bekanntmachung des Hinweises auf die Patenterteilung: **27.01.2010**
(21) Anmeldenummer: 07724464.8
(22) Anmeldetag: 23.04.2007
(51) Int. Cl.: G01N 33/98, C12Q 1/32

(54) **VERFAHREN UND MITTEL ZUR ENZYMATISCHEN BESTIMMUNG VON ETHANOL**
METHOD AND MEANS FOR THE ENZYMATIC DETERMINATION OF ETHANOL
PROCÉDÉ ET MOYEN DE DÉTERMINATION ENZYMATIQUE D'ÉTHANOL

(30) Priorität: 22.05.2006 DE 102006023897
(43) Veröffentlichungstag der Anmeldung: 11.02.2009
(73) Patentinhaber: Merck Patent GmbH, 64293 Darmstadt (DE)
(72) Erfinder: OLT, Ralf, 64750 Luetzelbach-Seckmauern (DE)
(86) Internationale Anmeldenummer: PCT/EP2007/003534
(87) Internationale Veröffentlichungsnummer: WO 2007/134683

(56) Entgegenhaltungen:
- EP-A- 0 464 942
- US-A- 3 926 736
- BEUTLER H O ET AL: "Neue Methode zur enzymatischen Bestimmung von Athanol in Lebensmitteln." ZEITSCHRIFT FUER ANALYTISCHE CHEMIE 1977 BOEHRINGER MANNHEIM GMBH, BIOCHEMICA WERKE TUTZING, POSTFACH 120, D-8132 TUTZING, FEDERAL REPUBLIC OF GERMANY, Bd. 284, Nr. 2, 1977, Seite 113, XP009086884

## Beschreibung

Die Erfindung betrifft ein Verfahren und ein Mittel zur enzymatischen Bestimmung von Ethanol. Das erfindungsgemäße Verfahren verhindert die Störung der Bestimmung durch Ethanoldämpfe in der Umgebungsluft.

Die Bestimmung von Ethanol spielt auf vielen Gebieten eine wichtige Rolle, wie z.B. in der Industrie zur Kontrolle von Fermentationsprozessen oder zur Bestimmung der Reinheit von Reagenzien, in der Lebensmittelindustrie zur Bestimmung des Alkoholgehaltes von Lebensmitteln und Getränken oder in der Medizin zur Bestimmung des Blutalkoholspiegels.

Der Alkoholgehalt der Proben kann dabei sehr unterschiedlich sein. Während bei der Bestimmung des Blutalkoholgehaltes geringe Mengen an Alkohol nachgewiesen werden müssen, muss z.B. bei alkoholischen Getränken ein hoher Ethanolgehalt genau bestimmt werden können.

Im Stand der Technik sind viele Bestimmungsmethoden bekannt. Teilweise ist für die Durchführung dieser Methoden ein großer technischer Aufwand nötig. Demgegenüber stehen enzymatische Bestimmungsmethoden, die schnell und einfach durchgeführt werden können. Diese Methoden können nasschemisch oder trockenchemisch, d.h. mit Hilfe eines Teststäbchens, durchgeführt werden. Ein Überblick über die wichtigsten Methoden zur Bestimmung von Ethanol findet sich in EP 0 164 008 auf Seite 2.

EP 0 164 008 beschäftigt sich weiterhin mit einer trockenchemischen enzymatischen Bestimmung von Ethanol, bei der das in einer Probe enthaltene Ethanol mit einer Alkoholoxidase in Gegenwart von Sauerstoff umgesetzt wird. Das entstehende Wasserstoffperoxid ruft dann mit Peroxidase und einem chromogenen Substrat eine sichtbare Färbung hervor, deren Intensität zur Bestimmung der Alkoholkonzentration vermessen werden kann.

Eine weitere Methode zur enzymatischen Bestimmung von Ethanol wird in H.-O. Beutler (Ethanol, in H.U. Bergmeyer (ed.), Methods of Enzymatic Analysis, 3rd Edition, Weinheim 1984, Vol. 6, Seiten 598 - 606) offenbart. Hier wird Ethanol in Anwesenheit von Alkoholdehydrogenase (ADH) und NAD zu Acetaldehyd umgesetzt. Acetaldehyd wiederum wird mittels NAD und Aldehyddehydrogenase zu Acetat umgesetzt. In beiden Reaktionsschritten entsteht NADH. Das gebildete NADH kann in bekannter Weise unter Reduktion eines Tetrazoliumsalzes zu einem gefärbten Formazan umgesetzt werden.

Auch in DD 256 196 A1 und US 5,294,540 werden trockenchemische Verfahren zur Bestimmung des Ethanolgehalts in Körperflüssigkeiten offenbart, in denen Ethanol in Anwesenheit von Alkoholdehydrogenase (ADH) und NAD zu Acetaldehyd umgesetzt wird.

Diese Verfahren haben, vor allem wenn sie auf Teststäbchen durchgeführt werden, den Nachteil, dass in der Umgebungsluft enthaltener Alkohol mit dem Indikatorsystem reagiert und die Messergebnisse verfälscht. Diese Störung spielt insbesondere in der Industrie bei der Kontrolle von Fermentationsprozessen, bei der Bestimmung der Reinheit von Reagenzien und in der Lebensmittelindustrie zur Bestimmung des Alkoholgehaltes von Lebensmitteln und Getränken eine wichtige Rolle. Verlässliche Resultate werden mit Alkohol-Teststreifen nur erhalten, wenn besondere Vorkehrungen zum Ausschluss dieser Störungen aus der Umgebungsluft getroffen werden.

Aufgabe der vorliegenden Erfindung war es, ein Verfahren zu entwickeln, mit dem eine Ethanolbestimmung so durchgeführt werden kann, dass eine Störung durch Ethanoldämpfe aus der Umgebungsluft minimiert oder ganz ausgeschlossen werden kann.

Es wurde gefunden, dass es möglich ist, die Störung der Bestimmung durch Ethanoldämpfe aus der Umgebungsluft zu eliminieren, wenn die Reaktion in zwei Schritten durchgeführt wird, wobei im ersten Schritt die enzymatische Umsetzung des Ethanols in Anwesenheit von Alkoholdehydrogenase (ADH) und NAD erfolgt, und in einem zweiten Schritt die reflektometrische bzw. colorimetrische Bestimmung des gebildeten Reaktionsprodukts NADH erfolgt, wobei im zweiten Schritt gleichzeitig die enzymatische Aktivität der Alkoholdehydrogenase unterdrückt wird.

Gegenstand der vorliegenden Erfindung ist daher ein Verfahren zur Bestimmung des Ethanolgehalts einer Probe gekennzeichnet durch folgende Verfahrensschritte:
a) Bereitstellung einer Probe deren Ethanolgehalt bestimmt werden soll
b) Kontaktieren der Probe mit einer Reagenzzusammenstellung A, die zumindest Alkoholdehydrogenase, NAD(P) und einen Puffer, der einen pH-Wert von größer oder gleich pH 7,5 erzeugt, enthält sowie entweder eine Verbindung, die mit Acetaldehyd eine Schiffsche Base bilden kann, oder Aldehyddehydrogenase (AIDH)
c) Inkubation der Mischung aus Schritt b)
d) Kontaktieren der Mischung aus Schritt c) mit einer Reagenzzusammenstellung B, die zumindest Reagenzien zur colorimetrischen Bestimmung von NAD(P)H enthält und Alkoholdehydrogenase inhibiert
e) Inkubation der Mischung aus Schritt d)
f) Bestimmung des Ethanolgehalts anhand der entstehenden Farbentwicklung

In einer bevorzugten Ausführungsform ist die Reagenzzusammenstellung A aus Schritt b) eine Reagenztablette.

In einer bevorzugten Ausführungsform ist die Reagenzzusammenstellung B aus Schritt d) ein Teststäbchen.

In einer bevorzugten Ausführungsform enthält die Reagenzzusammenstellung A aus Schritt b) NAD.

In einer bevorzugten Ausführungsform enthält die Reagenzzusammenstellung B aus Schritt d) 4-Jodpyrazol als Inhibitor der Alkoholdehydrogenase.

In einer bevorzugten Ausführungsform enthält die Reagenzzusammenstellung B aus Schritt d) zumindest Nitroblautetrazoliumchlorid und Diaphorase als Reagenzien zum colorimetrischen Nachweis von NAD(P)H.

In einer anderen bevorzugten Ausführungsform enthält die Reagenzzusammenstellung B aus Schritt d) zumindest Nitroblautetrazoliumchlorid und 1-Methoxy-5-methylphenazinium methylsulfat als Reagenzien zur colorimetrischen Bestimmung von NAD(P)H und einen Puffer, der einen pH-Wert von kleiner oder gleich 6 erzeugt.

In einer bevorzugten Ausführungsform erfolgt die Bestimmung des Ethanolgehaltes in Schritt f) reflektometrisch.

Gegenstand der vorliegenden Erfindung ist auch ein Testsatz zur Bestimmung des Ethanolgehalts einer Probe, zumindest enthaltend
a) eine Reagenzzusammenstellung A, die zumindest Alkoholdehydrogenase, NAD(P) und einen Puffer, der einen pH-Wert von größer oder gleich pH 7,5 erzeugt, enthält, sowie entweder eine Verbindung, die mit Acetaldehyd eine Schiffsche Base bilden kann, oder Aldehyddehydrogenase (AIDH) und
b) eine Reagenzzusammenstellung B, die zumindest Reagenzien zur colorimetrischen Bestimmung von NAD(P)H enthält und die Alkoholdehydrogenase inhibiert.

In einer bevorzugten Ausführungsform enthalten beide Reagenzzusammenstellungen unabhängig voneinander Puffer und Stabilisatoren.

In einer bevorzugten Ausführungsform liegt die Reagenzzusammenstellung A in Form einer Reagenztablette vor und die Reagenzzusammenstellung B in Form eines Teststäbchens.

In der vorliegenden Schrift werden die folgenden Abkürzungen verwendet:
- NAD: Nicotinamidadenindinucleotid (oxidierte Form)
- NAD(P): Nicotinamidadenindinucleotid und/oder Nicotinamidadenindinucleotidphosphat (oxidierte Form)
- NAD(P)H: Nicotinamidadenindinucleotid und/oder Nicotinamidadenindinucleotidphosphat (reduzierte Form)
- NADH: Nicotinamidadenindinucleotid (reduzierte Form)
- NADP: Nicotinamidadenindinucleotidphosphat (oxidierte Form)
- NADPH: Nicotinamidadenindinucleotidphosphat (reduzierte Form)
- NBT: Nitroblautetrazoliumchlorid

Eine Probe ist erfindungsgemäß typischerweise flüssig. Die Probe kann beispielsweise eine Lösung oder Suspension sein, die in der Regel als vorwiegendes Lösungsmittel Wasser oder eine Pufferlösung enthält. Feste Proben werden zunächst in Wasser oder einer Pufferlösung gelöst, suspendiert oder damit extrahiert. Beispiele für Proben sind verdünnte Säfte, alkoholische Getränke oder Blut. Je nach Beschaffenheit der Probe und deren Alkoholgehalt kann es vorteilhaft sein, die Probe vor der Durchführung der erfindungsgemäßen Bestimmung mit Wasser oder einer Pufferlösung zu verdünnen.

Eine colorimetrische Bestimmung ist erfindungsgemäß eine Bestimmung, bei der ein im visuellen Bereich sichtbar gefärbtes Produkt entsteht, das visuell, bevorzugt halbquantitativ bzw. quantitativ ausgewertet werden kann. Bevorzugt erfolgt die Auswertung reflektometrisch, z.B. mit einfachen Reflektometern.

In jüngerer Zeit hat die Analyse mit festen, saugfähigen Trägern, den sogenannten Teststäbchen, zunehmend an Bedeutung gewonnen. Zu den wesentlichen Vorteilen dieser trockenchemischen Verfahren gehören insbesondere die einfache Handhabung sowie die aufgrund der geringen Reagenzmengen unproblematische Entsorgung. Alle oder der größte Teil der für die Nachweisreaktion notwendigen Reagenzien sind dabei in entsprechenden Schichten eines festen, saugfähigen oder quellbaren Trägers eingebettet, auf den die Probe aufgebracht wird. Nach Kontakt der Reaktionszone mit der Probe läuft die Nachweisreaktion ab. Die gebildete Farbe ist ein Maß für die Menge des zu bestimmenden Analyten und kann visuell, d.h. halbquantitativ, bzw. quantitativ mit einfachen Reflektometern ausgewertet werden.

Als saugfähige Träger für die Teststreifen des erfindungsgemäßen Testsatzes können alle Materialien verwendet werden, die üblicherweise für solche trockenchemischen Tests im Gebrauch sind. Am weitesten verbreitet ist die Verwendung von Filterpapier, jedoch können auch andere saugfähige Cellulose- oder Kunststoffprodukte eingesetzt werden.

Die saugfähigen Träger werden in bekannter Weise mit Tränklösungen imprägniert. Bevorzugt enthalten die Tränklösungen alle zur Bestimmung des Analyten notwendigen Reagenzien.

Die getränkten und getrockneten Träger können geeignet zugeschnitten und in bekannter Weise zur Herstellung der Teststreifen auf Trägerfolien aufgeklebt bzw. aufgesiegelt werden.

Zumeist bedeckt der saugfähige Träger, auf den die Nachweisreagenzien aufgebracht sind, nicht den gesamten Teststreifen, sondern lediglich eine Zone des Teststreifens. Auf diese Weise ist es möglich, nicht nur eine Zone mit einer Zusammensetzung von Nachweisreagenzien, sondern mehrere Zonen mit gleicher oder unterschiedlichen Zusammensetzungen auf einem Teststreifen zu kombinieren. Erfindungsgemäß wird daher der Bereich des Teststreifens, auf den die für die Bestimmung eines Analyten oder zur Erstellung eines Leerwerts notwendigen Reagenzien auf einen saugfähigen Träger aufgebracht sind, als Zone bezeichnet.

Die erfindungsgemäße Bestimmung des Ethanolgehalts gemäß Anspruch 1 erfolgt in zwei Schritten. Zunächst erfolgt eine Umsetzung des Ethanols in der Probe mit Alkoholdehydrogenase und NAD(P) (Verfahrensschritte a) bis c) des erfindungsgemäßen Verfahrens). Dabei wird das Ethanol zu Acetaldehyd umgesetzt, während NAD(P) zu NAD(P)H reduziert wird. Dieser Reaktionsschritt wird erfindungsgemäß in Lösung durchgeführt. Dazu wird die Probe mit einer Reagenzzusammenstellung A gemischt oder in Kontakt gebracht, die zumindest Alkoholdehydrogenase, NAD(P) und einen Puffer enthält, der einen pH-Wert von größer oder gleich pH 7,5 erzeugt, sowie entweder eine Verbindung, die mit Acetaldehyd eine Schiffsche Base bilden kann, oder Aldehyddehydrogenase (AIDH).

Durch die Anwesenheit von Aldehyddehydrogenase oder der Verbindung, die mit Acetaldehyd eine Schiffsche Base bilden kann, wird Acetaldehyd aus dem Reaktionsgemisch entfernt und so das Gleichgewicht der Reaktion in Richtung Acetaldehyd-Bildung verschoben. Nähere Angaben zum Einsatz von Aldehyddehydrogenase bei dieser Umsetzung von Ethanol finden sich in H.-O-Beutler (Ethanol, in H.U. Bergmeyer (ed.), Methods of Enzymatic Analysis, 3rd Edition, Weinheim 1984, Vol. 6, Seiten 598 - 606, insbesondere auf den Seiten 601-603).

Bevorzugt enthält die Reagenzzusammenstellung A zumindest Alkoholdehydrogenase und NAD, sowie eine Verbindung, die mit Acetaldehyd eine Schiffsche Base bilden kann und einen Puffer, der einen pH-Wert von größer oder gleich pH 7,5 erzeugt.

Puffersubstanzen, die einen pH-Wert größer oder gleich pH 7,5 erzeugen können, sind dem Fachmann bekannt. Bevorzugt wird erfindungsgemäß TRIS Puffer (Tris(hydroxymethyl)aminomethan eingesetzt, da TRIS einen geeigneten basischen pH-Wert erzeugen kann und zugleich eine Verbindung darstellt, die mit Acetaldehyd eine Schiffsche Base bilden kann.

Bevorzugt enthält die Reagenzzusammenstellung A Puffersubstanzen, die einen pH-Wert zwischen 8 und 10 erzeugen.

Verbindungen, die mit Acetaldehyd eine Schiffsche Base bilden können sind insbesondere Amine, Hydrazine, Semicarbazide oder Hydroxylamine. Bevorzugt werden Hydrazin oder Hydroxylamin, besonders bevorzugt TRIS eingesetzt.

Da TRIS wie oben erläutert sowohl geeignet ist, einen pH-Wert größer oder gleich pH 7,5 zu erzeugen als auch mit Acetaldehyd eine Schiffsche Base bilden kann, wird TRIS besonders bevorzugt eingesetzt. In diesem Fall erfüllt eine Verbindung, nämlich TRIS, beide Anforderungen.

Alkoholdehydrogenase und NAD(P) liegen in so ausreichender Menge vor, dass eine zügige und vollständige Umsetzung des Ethanols gewährleistet ist.

Die Reagenzzusammenstellung A kann in flüssiger Form als Lösung oder in fester Form z.B. als Pulver, Kapsel oder Reagenztablette vorliegen. Bevorzugt liegt die Reagenzzusammenstellung als Tablette vor. Typischerweise enthält die Reagenzzusammenstellung A weitere Bestandteile wie Puffersubstanzen, Stabilisatoren oder gegebenenfalls Tablettierhilfsmittel.

Liegt Reagenzzusammenstellung A als Reagenztablette vor, kann sie beispielsweise wie folgt zusammengesetzt sein:

| | | |
|---|---|---|
| 25-30 | mg | Tris(hydroxymethyl-)aminomethan |
| 1-1,8 | mg | Glycin |
| 10-15 | U | Alkoholdehydrogenase |
| 5-6 | mg | Nicotinamid-adenin-dinucleotid |
| Füllstoffe und Stabilisatoren | | |

Bevorzugt ist die Reagenztablette wie folgt zusammengesetzt:

| | | |
|---|---|---|
| 28,4 | mg | Tris(hydroxymethyl-)aminomethan |
| 1,42 | mg | Glycin |
| 13,4 | U | Alkoholdehydrogenase |
| 5,58 | mg | Nicotinamid-adenin-dinucleotid |
| ad 67,5 | mg | Füllstoffe und Stabilisatoren |

Um eine möglichst vollständige Umsetzung des Ethanols in der Probe zu gewährleisten, wird die Mischung von Probe und Reagenzzusammenstellung A inkubiert. Die Inkubationszeit liegt typischerweise zwischen 30 Sekunden und 10 Minuten. Bevorzugt erfolgt die Inkubation bei Temperaturen zwischen 18 und 37 °C, besonders bevorzugt bei Raumtemperatur.

In H.-O-Beutler (Ethanol, in H.U. Bergmeyer (ed.), Methods of Enzymatic Analysis, 3rd Edition, Weinheim 1984, Vol. 6, Seiten 598 - 606) wird ausführlich beschrieben, wie die Bestimmung von Ethanol mit Alkoholdehydrogenase und NAD(P) durchgeführt werden kann.

Anschließend wird erfindungsgemäß in einem zweiten Schritt (Verfahrensschritte d) bis f) des erfindungsgemäßen Verfahrens) die Konzentration des entstandenen NAD(P)H bestimmt. Dies erfolgt durch Mischen oder Inkontaktbringen der aus Verfahrensschritt c) resultierenden Mischung mit einer Reagenzzusammenstellung B, die zumindest Reagenzien zum colorimetrischen Nachweis von NAD(P)H enthält und Alkoholdehydrogenase inhibiert. Die Inhibierung der Alkoholdehydrogenase kann dabei durch Zusatz eines Inhibitors zu der Reagenzzusammenstellung B erfolgen, oder indem die Reagenzzusammenstellung B einen pH-Wert erzeugt, der Alkoholdehydrogenase inaktiviert.

Die Reagenzzusammenstellung B kann in flüssiger Form als Lösung, in fester Form, z.B. als Pulver, Kapsel oder Reagenztablette, oder als Teststäbchen vorliegen. Bevorzugt liegt die Reagenzzusammenstellung als Teststäbchen vor. Dabei sind bevorzugt alle für die Bestimmung von NAD(P)H notwendigen Reagenzien auf dem Teststäbchen enthalten.

Typischerweise enthält die Reagenzzusammenstellung B Reagenzien zur colorimetrischen Bestimmung von NAD(P)H, einen Inhibitor der Alkoholdehydrogenase oder Puffersubstanzen zur Erzeugung eines pH-Wertes, der die Alkoholdehydrogenase inaktiviert sowie optionale weitere Bestandteile wie Puffersubstanzen, Stabilisatoren oder Tablettierhilfsmittel.

Erfolgt die colorimetrische Bestimmung von NAD(P)H enzymatisch mittels Diaphorase unter Reduktion eines Tetrazoliumsalzes zu einem gefärbten Formazan, so sollte diese Umsetzung bei einem pH-Wert zwischen 7 und 9 erfolgen, um eine ausreichende Aktivität der Diaphorase zu gewährleisten. Bei pH-Werten zwischen 7 und 9 ist jedoch auch die Alkoholdehydrogenase aktiv, so dass in diesem Fall erfindungsgemäß der Reagenzzusammenstellung b) ein Inhibitor der Alkoholdehydrogenase zugesetzt wird.

Als Inhibitoren für die Alkoholdehydrogenase können alle bekannten Alkoholdehydrogenase-Inhibitoren verwendet werden, die den Nachweis von NAD(P)H nicht stören. Dies sind beispielsweise Pyrazol, 4-Alkylpyrazole und in Position 4 halogenierte Pyrazole, vorzugsweise 4-Jodpyrazol.

Sowohl das Enzym Diaphorase wie auch das Tetrazoliumsalz und der Inhibitor der Alkoholdehydrogenase werden in solchen Konzentrationen eingesetzt, dass eine zügige Umsetzung erfolgt. Eine zügige Umsetzung bedeutet vorzugsweise Reaktionszeiten von weniger als 15 Minuten.

In einer bevorzugten Ausführungsform wird das in Verfahrensschritt b) bzw. c) gebildete NAD(P)H nicht enzymatisch sondern mittels eines Stoffes wie Phenazinmethosulfat oder Phenazinethosulfat oder Derivaten davon, wie z.B. vorzugsweise 1-Methoxy-5-methylphenazinium methylsulfat (MPMS) unter Reduktion eines Tetrazoliumsalzes zu einem gefärbten Formazan umgesetzt. In diesem Fall kann die Inhibierung der Alkoholdehydrogenase mittels eines Inhibitors erfolgen oder durch Veränderung des pH-Wertes in einen Bereich, in dem Alkoholdehydrogenase nicht mehr aktiv ist. Bevorzugt erfolgt die Inhibierung der Alkoholdehydrogenase durch Veränderung des pH-Wertes. Die Inhibierung der Alkoholdehydrogenase ist bei Einstellung eines pH-Wertes unter pH 6, bevorzugt zwischen 2 und 5,5 besonders effektiv.

In einer besonders bevorzugten Ausführungsform enthält Reagenzzusammenstellung b) daher zumindest Phenazinmethosulfat, Phenazinethosulfat oder ein Derivat davon (vorzugsweise 1-Methoxy-5-methylphenazinium methylsulfat *(MPMS))* und ein Tetrazoliumsalz, so dass das gebildete NAD(P)H durch Phenazinmethosulfat oder einem Derivat davon unter Reduktion des Tetrazoliumsalzes zu einem gefärbten Formazan umgesetzt wird. Weiterhin enthält die Reagenzzusammenstellung B einen Puffer, der einen pH-Wert kleiner oder gleich pH 6, bevorzugt zwischen pH 2,0 und pH 5,5, besonders bevorzugt ca. pH 3,5, einstellt. Im Falle eines Teststäbchens sollte dieses zumindest in der Nachweiszone einen Puffer enthalten, der für die Bestimmungsreaktion die oben genannten pH-Werte einstellt.

Als Puffer sind alle Puffersubstanzen geeignet, die im pH-Bereich unterhalb von ca. pH 6, bevorzugt zwischen pH 2,0 und 5,5 eine puffernde Wirkung entfalten, z.B. Zitronensäure und ihre Salze, Äpfelsäure und ihre Salze, Bernsteinsäure und ihre Salze, Weinsäure und ihre Salze, Diglykolsäure und ihre Salze, vorzugsweise Zitronensäure/Citrat. Die Konzentration des Puffers in der Tränklösung wird so gewählt, dass die vom Papier aufgenommene Probe von z.B. pH 9,5 durch den im Papier enthaltenen Puffer auf einen pH von z.B. < 5,0 umgepuffert wird, z.B. 425 mmol/l Zitronensäure/Citrat bei pH 3,5.

Geeignete Tetrazoliumsalze sind z.B. NBT oder MTT (3-(4,5-Dimethylthiazol-2-yl)-2,5-diphenyl-2-tetrazoliumbromid).

Um eine möglichst vollständige Umsetzung des NAD(P)H zu gewährleisten, wird nach Inkontaktbringen mit Reagenzzusammenstellung B inkubiert. Die Inkubationszeit liegt typischerweise zwischen 30 Sekunden und 10 Minuten. Bevorzugt erfolgt die Inkubation bei Temperaturen zwischen 18 und 37 °C , besonders bevorzugt bei Raumtemperatur.

Nach dem Ende der Reaktion wird die Intensität der Farbe des Formazans bestimmt. Dies kann visuell erfolgen, z.B. halbquantitativ durch einen Vergleich mit einer Farbkarte oder quantitativ z.B. mittels eines Photometers oder Reflektometers. Bei Einsatz eines Teststreifens wird bevorzugt reflektometrisch die Remission gemessen. Über eine Kalibrationskurve kann hieraus die Konzentration des Ethanols in der Probe berechnet werden.

Gegenstand der vorliegenden Erfindung ist auch ein Testsatz gemäß Anspruch 9 zur Durchführung des erfindungsgemäßen Verfahrens.
Der Testsatz enthält zumindest
a) eine Reagenzzusammenstellung A, die zumindest Alkoholdehydrogenase, NAD(P) und einen Puffer, der einen pH-Wert von größer oder gleich pH 7,5 erzeugt, enthält sowie entweder eine Verbindung, die mit Acetaldehyd eine Schiffsche Base bilden kann, oder Aldehyddehydrogenase (AIDH) und
b) eine Reagenzzusammenstellung B, die zumindest Reagenzien zur colorimetrischen Bestimmung von NAD(P)H enthält und Alkoholdehydrogenase inhibiert.

Bezüglich der Zusammensetzung der Reagenzzusammenstellungen und der bevorzugten Ausführungsformen wird auf die Ausführungen bei der Beschreibung des erfindungsgemäßen Verfahrens verwiesen.

In einer bevorzugten Ausführungsform enthält die
Reagenzzusammenstellung A einen Puffer, der einen pH-Wert zwischen pH 8 und 10 erzeugt.
In einer besonders bevorzugten Ausführungsform ist der Puffer ein Amin-Puffer, wie z.B. TRIS.

In einer bevorzugten Ausführungsform liegt Reagenzzusammenstellung A in Form einer Reagenztablette vor und Reagenzzusammenstellung B in Form eines Teststäbchens.

Es wurde gefunden, dass bei Einsatz des erfindungsgemäßen Verfahrens und Testsatzes der störende Einfluss von Ethanol aus der Umgebungsluft vermieden werden kann. "Vermieden werden" bedeutet dabei erfindungsgemäß, dass bei vergleichenden Messungen mit und ohne Ethanol in der Umgebungsluft kein signifikanter Unterschied im Messergebnis auftritt.

Somit bieten das erfindungsgemäße Verfahren und der erfindungsgemäße Testsatz eine einfache, schnelle und empfindliche Methode zur enzymatischen Bestimmung von Ethanol. Durch die bevorzugte Verfahrensdurchführung mithilfe eines Teststreifens kann die Bestimmung ohne apparativen Aufwand und ohne Handhabung von giftigen Chemikalien auch von ungeübtem Personal durchgeführt werden.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

### Beispiele

### Beispiel 1:

Es werden Reagenztabletten für die Ethanolbestimmung mit folgender Zusammensetzung hergestellt:

| | | | |
|---|---|---|---|
| | 28,4 | mg | Tris(hydroxymethyl-)aminomethan |
| | 1,42 | mg | Glycin |
| | 13,4 | U | Alkoholdehydrogenase |
| | 5,58 | mg | Nicotinamid-adenin-dinucleotid |
| ad | 67,5 | mg | Füllstoffe und Stabilisatoren |

Es werden Teststreifen mit zwei Papierzonen hergestellt, wobei auf beide Zonen eine Lösung folgender Zusammensetzung aufgetragen wird:

| | | |
|---|---|---|
| 50 | mmol/L | Triethanolamin-Puffer pH 7,2 |
| 1 | g/L | Nitroblautetrazoliumchlorid |
| 85 | U/I | Diaphorase |
| 1 | mol/I | 4-Jodpyrazol |

Nach dem Trocknen werden diese Teststreifen bei 2°C bis 8°C trocken gelagert.

Die Bestimmung wird wie folgt durchgeführt:

Eine Reagenztablette wird in ein verschließbares Reagenzgefäß vorgelegt und in 0,5 ml vorverdünnter Probe gelöst. Die Lösung wird bei Raumtemperatur 4 Minuten inkubiert. Anschließend wird die Lösung mit 10 ml Wasser verdünnt, und der Teststreifen wird eingetaucht. Überschüssige Flüssigkeit wird von dem Teststreifen entfernt, und der Teststreifen wird 3 Minuten bei Raumtemperatur inkubiert. Die gebildete Farbe des Formazans wird reflektometrisch gemessen, und über eine Kalibrationskurve wird die Ethanolkonzentration berechnet.

### Beispiel 2:

Es werden Reagenztabletten für die Ethanolbestimmung mit folgender Zusammensetzung hergestellt:

| | | | |
|---|---|---|---|
| | 28,4 | mg | Tris(hydroxymethyl-)aminomethan |
| | 1,42 | mg | Glycin |
| | 13,4 | U | Alkoholdehydrogenase |
| | 5,58 | mg | Nicotinamid-adenin-dinucleotid |
| ad | 67,5 | mg | Füllstoffe und Stabilisatoren |

Es werden Teststreifen mit zwei Papierzonen hergestellt, wobei auf beide Zonen eine Lösung folgender Zusammensetzung aufgetragen wird:

| | | |
|---|---|---|
| 425 | mmol/L | Citrat-Puffer pH 3,5 |
| 1 | g/L | Nitroblautetrazoliumchlorid |
| 6 | µmol/l | 1-Methoxy-5-methylphenazinium methylsulfat |

Nach dem Trocknen werden diese Teststreifen bei 2°C bis 8°C trocken gelagert.

Die Bestimmung wird wie folgt durchgeführt:

Eine Reagenztablette wird in ein verschließbares Reagenzgefäß vorgelegt und in 0,5 ml vorverdünnter Probe gelöst. Die Lösung wird bei Raumtemperatur 4 Minuten inkubiert. Anschließend wird die Lösung mit 10 ml Wasser verdünnt, und der Teststreifen wird eingetaucht. Überschüssige Flüssigkeit wird von dem Teststreifen entfernt, und der Teststreifen wird 3 Minuten bei Raumtemperatur inkubiert. Die gebildete Farbe des Formazans wird reflektometrisch gemessen, und über eine Kalibrationskurve wird die Ethanolkonzentration berechnet.

## Patentansprüche

1. Verfahren zur Bestimmung des Ethanolgehalts einer Probe **gekennzeichnet durch** folgende Verfahrensschritte:
a) Bereitstellung einer Probe deren Ethanolgehalt bestimmt werden soll
b) Kontaktieren der Probe mit einer Reagenzzusammenstellung A enthaltend zumindest Alkoholdehydrogenase, NAD(P) und einen Puffer, der einen pH-Wert von größer oder gleich pH 7,5 erzeugt, sowie entweder eine Verbindung, die mit Acetaldehyd eine Schiffsche Base bilden kann, oder Aldehyddehydrogenase (AIDH)
c) Inkubation der Mischung aus Schritt b)
d) Kontaktieren der Mischung aus Schritt c) mit einer Reagenzzusammenstellung B, die zumindest Reagenzien zur colorimetrischen Bestimmung von NAD(P)H enthält und Alkoholdehydrogenase inhibiert
e) Inkubation der Mischung aus Schritt d)
f) Bestimmung des Ethanolgehalts anhand der entstehenden Farbentwicklung

2. Verfahren nach Anspruch 1, **dadurch gekennzeichnet, dass** die Reagenzzusammenstellung A aus Schritt b) eine Reagenztablette ist.

3. Verfahren nach Anspruch 1 oder 2, **dadurch gekennzeichnet, dass** die Reagenzzusammenstellung B aus Schritt d) ein Teststäbchen ist.

4. Verfahren nach einem oder mehreren der Ansprüche 1 bis 3, **dadurch gekennzeichnet, dass** die Reagenzzusammenstellung A aus Schritt b) NAD enthält.

5. Verfahren nach einem oder mehreren der Ansprüche 1 bis 4, **dadurch gekennzeichnet, dass** die Reagenzzusammenstellung B aus Schritt d) 4-Jodpyrazol als Inhibitor der Alkoholdehydrogenase enthält.

6. Verfahren nach einem oder mehreren der Ansprüche 1 bis 5, **dadurch gekennzeichnet, dass** die Reagenzzusammenstellung B aus Schritt d) zumindest Nitroblautetrazoliumchlorid und Diaphorase als Reagenzien zum colorimetrischen Nachweis von NAD(P)H enthält.

7. Verfahren nach einem oder mehreren der Ansprüche 1 bis 5, **dadurch gekennzeichnet, dass** die Reagenzzusammenstellung B aus Schritt d) zumindest Nitroblautetrazoliumchlorid und 1-Methoxy-5-methylphenazinium methylsulfat als Reagenzien zur colorimetrischen Bestimmung von NAD(P)H enthält und einen Puffer, der einen pH-Wert von kleiner oder gleich 6 erzeugt.

8. Verfahren nach einem oder mehreren der Ansprüche 1 bis 7, **dadurch gekennzeichnet, dass** die Bestimmung des Ethanolgehaltes in Schritt f) reflektometrisch erfolgt.

9. Testsatz zur Bestimmung des Ethanolgehalts einer Probe, zumindest enthaltend
a) eine Reagenzzusammenstellung A, die zumindest Alkoholdehydrogenase, NAD(P) und einen Puffer enthält, der einen pH-Wert von größer oder gleich pH 7,5 erzeugt, sowie entweder eine Verbindung, die mit Acetaldehyd eine Schiffsche Base bilden kann, oder Aldehyddehydrogenase (AIDH) und
b) eine Reagenzzusammenstellung B, die zumindest Reagenzien zur colorimetrischen Bestimmung von NAD(P)H enthält und die Alkoholdehydrogenase inhibiert.

10. Testsatz nach Anspruch 9, **dadurch gekennzeichnet, dass** beide Reagenzzusammenstellungen unabhängig voneinander Puffer und Stabilisatoren enthalten.

11. Testsatz nach Anspruch 9 oder 10, **dadurch gekennzeichnet, dass** die Reagenzzusammenstellung A in Form einer Reagenztablette vorliegt und die Reagenzzusammenstellung B in Form eines Teststäbchens.

## Claims

1. Method for the determination of the ethanol content of a sample, **characterised by** the following method steps:
a) provision of a sample whose ethanol content is to be determined
b) bringing the sample into contact with a reagent composition A comprising at least alcohol dehydrogenase, NAD(P) and a buffer which produces a pH of greater than or equal to pH 7.5, and either a compound which is able to form a Schiff base with acetaldehyde, or aldehyde dehydrogenase (AIDH)
c) incubation of the mixture from step b)
d) bringing the mixture from step c) into contact with a reagent composition B which comprises at least reagents for the colorimetric determination of NAD(P)H and inhibits alcohol dehydrogenase
e) incubation of the mixture from step d)
f) determination of the ethanol content from the colour development forming.

2. Method according to Claim 1, **characterised in that** the reagent composition A from step b) is a reagent tablet.

3. Method according to Claim 1 or 2, **characterised in that** the reagent composition B from step d) is a test stick.

4. Method according to one or more of Claims 1 to 3, **characterised in that** the reagent composition A from step b) comprises NAD.

5. Method according to one or more of Claims 1 to 4, **characterised in that** the reagent composition B from step d) comprises 4-iodopyrazole as inhibitor of alcohol dehydrogenase.

6. Method according to one or more of Claims 1 to 5, **characterised in that** the reagent composition B from step d) comprises at least nitro blue tetrazolium chloride and diaphorase as reagents for the colorimetric determination of NAD(P)H.

7. Method according to one or more of Claims 1 to 5, **characterised in that** the reagent composition B from step d) comprises at least nitro blue tetrazolium chloride and 1-methoxy-5-methylphenazinium methylsulfate as reagents for the colorimetric determination of NAD(P)H and a buffer which produces a pH of less than or equal to 6.

8. Method according to one or more of Claims 1 to 7, **characterised in that** the determination of the ethanol content in step f) is carried out by reflectometry.

9. Test set for the determination of the ethanol content of a sample, at least comprising
a) a reagent composition A which comprises at least alcohol dehydrogenase, NAD(P) and a buffer which produces a pH of greater than or equal to pH 7.5, and either a compound which is able to form a Schiff base with acetaldehyde, or aldehyde dehydrogenase (AIDH) and
b) a reagent composition B which comprises at least reagents for the colorimetric determination of NAD(P)H and inhibits alcohol dehydrogenase.

10. Test set according to Claim 9, **characterised in that** both reagent compositions comprise, independently of one another, buffers and stabilisers.

11. Test set according to Claim 9 or 10, **characterised in that** the reagent composition A is in the form of a reagent tablet and the reagent composition B is in the form of a test stick.

## Revendications

1. Méthode de détermination de la teneur en éthanol d'un échantillon, **caractérisée par** les étapes méthodologiques suivantes consistant à :
a) fournir un échantillon dont la teneur en éthanol est à déterminer,
b) amener l'échantillon en contact avec une composition de réactifs A comprenant au moins de l'alcool déshydrogénase, du NAD(P) et un tampon qui produit un pH supérieur ou égal à pH 7,5, et soit un composé capable de former une base de Schiff avec l'acétaldéhyde, soit de l'aldéhyde déshydrogénase (AIDH)
c) incuber le mélange issu de l'étape b)
d) amener le mélange issu de l'étape c) en contact avec une composition de réactifs B comprenant au moins des réactifs pour la détermination colorimétrique du NAD(P)H et inhibant l'alcool déshydrogénase
e) incuber le mélange issu de l'étape d)
f) déterminer la teneur en éthanol à partir du développement de couleur se formant.

2. Méthode selon la revendication 1, **caractérisée en ce que** la composition de réactifs A de l'étape b) est un comprimé de réactifs.

3. Méthode selon la revendication 1 ou 2, **caractérisée en ce que** la composition de réactifs B de l'étape d) est un bâtonnet réactif.

4. Méthode selon l'une ou plusieurs des revendications 1 à 3, **caractérisée en ce que** la composition de réactifs A de l'étape b) comprend du NAD.

5. Méthode selon l'une ou plusieurs des revendications 1 à 4, **caractérisée en ce que** la composition de réactifs B de l'étape d) comprend du 4-iodopyrazole comme inhibiteur de l'alcool déshydrogénase.

6. Méthode selon l'une ou plusieurs des revendications 1 à 5, **caractérisée en ce que** la composition de réactifs B de l'étape d) comprend au moins du chlorure de nitro-bleu de tétrazolium et de la diaphorase comme réactifs pour la détermination colorimétrique de NAD(P)H.

7. Méthode selon l'une ou plusieurs des revendications 1 à 5, **caractérisée en ce que** la composition de réactifs B de l'étape d) comprend au moins du chlorure de nitro-bleu de tétrazolium et du méthylsulfate de 1-méthoxy-5-méthylphénazinium comme réactifs pour la détermination colorimétrique de NAD(P)H et un tampon qui produit un pH inférieur ou égal à 6.

8. Méthode selon l'une ou plusieurs des revendications 1 à 7, **caractérisée en ce que** la détermination de la teneur en éthanol dans l'étape f) est effectuée par réflectométrie.

9. Kit d'essai destiné à la détermination de la teneur en éthanol d'un échantillon, comprenant au moins
a) une composition de réactifs A comprenant au moins de l'alcool déshydrogénase, du NAD(P) et un tampon qui produit un pH supérieur ou égal à pH 7,5, et un composé capable de former une base de Schiff avec de l'acétaldéhyde, ou bien de l'aldéhyde déshydrogénase (AIDH) et
b) une composition de réactifs B comprenant au moins des réactifs pour la détermination colorimétrique du NAD(P)H et inhibant l'alcool déshydrogénase.

10. Kit d'essai selon la revendication 9, **caractérisé en ce que** les deux compositions de réactifs comprennent, indépendamment l'une de l'autre, des tampons et des stabilisants.

11. Kit d'essai selon la revendication 9 ou 10, **caractérisé en ce que** la composition de réactifs A est sous la forme d'un comprimé de réactifs et la composition de réactifs B est sous la forme d'un bâtonnet réactif.
